(19) Europäisches Patentamt

European Patent Office

Office européen des brevets

(11) Publication number:. **0 313 158**
**A2**

(12) **EUROPEAN PATENT APPLICATION**

(21) Application number: 88202305.4

(22) Date of filing: 14.10.88

(51) Int. Cl.⁴: **C07K 7/06 , A61K 37/02**

Claims for the following Contracting State: ES.

(30) Priority: 23.10.87 US 111755

(43) Date of publication of application:
**26.04.89 Bulletin 89/17**

(84) Designated Contracting States:
**AT BE CH DE ES FR GB IT LI LU NL SE**

(71) Applicant: **MERCK & CO. INC.**
**126, East Lincoln Avenue P.O. Box 2000**
**Rahway New Jersey 07065-0900(US)**

(72) Inventor: **Oliff, Allen I.**
**1412 Florence Drive**
**Gwynedd Valley, PA 19437(US)**
Inventor: **Riemen, Mark W.**
**326 Pheasant Run Drive**
**Doylestown, PA 18901(US)**
Inventor: **Heimbrook, David C.**
**812 Sandra Road**
**RD No. 2, Ringoes New Jersey 08551(US)**

(74) Representative: **Hesketh, Alan, Dr.**
**European Patent Department Merck & Co.,**
**Inc. Terlings Park Eastwick Road**
**Harlow Essex, CM20 2QR(GB)**

(54) **Gastrin releasing peptide antagonist.**

(57) Small cell lung carcinoma cells (SCLC) contain gastrin releasing peptide (GRP) receptors. The response of the cells to GRP is rapid growth. We have found a group of heptapeptides that act as GRP antagonists by blocking the binding of GRP to its receptor thereby inhibiting the growth of cells that are sensitive to the growth promoting activity of GRP.

EP 0 313 158 A2

## GASTRIN RELEASING PEPTIDE ANTAGONIST

BACKGROUND OF THE INVENTION

Gastrin releasing peptide (GRP), a 27-amino acid hormone, stimulates the growth of small cell lung carcinoma (SCLC) cells in cell culture. Antibodies directed against GRP block the growth of SCLC in nude mice.

DISCLOSURE STATEMENT

Broccardo et al., Br. J. Pharmac. 55:221-227 (1975) compare the pharmacological activity of two natural bombesin-like peptides and 25 related synthetic peptides to that of bombesin. ·

Marki et al., Peptides 2, Suppl. 2:169-177 (1981) disclose structure activity relationship of 26 peptide analogs of bombesin and GRP. The minimal essential residues required for full potency of bombesin-like effects is represented by an acetylated C-terminal 8-peptide fragment wherein position 7 can be substituted by alanine, histidine, glutamine or D-glutamine. Modification of the tryptophan [8] and histidine [12] residues by alanine abolished the biological potency of these peptides. A blocked N-terminus is necessary for maximum response.

Moody et al., Peptides 4 (5):683-686 (1983) disclose the presence of high concentrations of bombesin-like peptides and receptors in small cell lung cancer (SCLC) and suggest that bombesin may function as an important regulatory agent in human SCLC.

Jensen et al., Nature 309:61-63 (3 May 1984) diclose that a substance P analog is also a bombesin receptor antagonist.

Weber et al., J. Clin. Invest. 75:306-309 (1985) disclose that the mitogenicity of gastrin releasing peptide (GRP) resides in its carboxy terminal fragment, designated GRP 14-27, which is partly homologous to bombesin. The authors speculate that GRP or a closely related small peptide may be acting as an autocrine growth factor for SCLC.

Cuttitta et al., Nature, 316:823-826 (29 August 1985) disclose that a monoclonal antibody to bombesin blocks the binding of the hormone to cellular receptors and inhibits the clonal growth of SCLC in vitro and the growth of SCLC xenografts in vivo demonstrating that bombesin-like peptides can function as autocrine growth factors for human SCLC.

Corps et al., Biochem. J. 231:781-784 (1985) disclose that an analog of substance P inhibits the stimulation of DNA synthesis induced in Swiss 3T3 cells by bombesin.

Bepler et al., Cancer Research 47:2371-2375 (1 May 1987) disclose that the undecapeptide physalaemin inhibits the clonal and mass culture growth of SCLC cell lines at picomolar concentrations.

Heinz-Erian et al., Am. J. Physiol. 252:G439-G442 (1987) disclosed that [D-Phe[12]] analogs of bombesin are the only bombesin receptor antagonists identified to date that interact only with the bombesin receptor.

OBJECTS OF THE INVENTION

It is an object of the present invention to provide small peptides that act as antagonists of GRP. Another object of the present invention is to provide fragments of GRP that act as antagonists of GRP. A further object is to provide a method of treating SCLC by administering a peptide of the present invention. These and other objects of the present invention will be apparent from the following description.

SUMMARY OF THE INVENTION

A series of heptapeptides have been found which are GRP antagonists and which suppress GRP stimulated mitogenesis in Swiss 3T3 cells.

The peptides of the present invention are the following:

$$His \quad Trp \quad Ala \quad Val \quad Gly \quad His \quad Leu-NH_2$$

$$Ac-His \quad Trp \quad Ala \quad Val \quad Gly \quad His \quad Leu-NH_2$$

$$Ac-His \quad Trp \quad Ala \quad Val \quad Gly \quad His \quad Leu-NHR^1$$

$$Ac-His \quad Trp \quad Ala \quad Val \quad Gly \quad His \quad Leu-OR^1$$

wherein $R^1$ is a straight or branched chain alkyl group of from 1 to 5 carbon atoms, or a phenyl group either of which is optionally substituted by alkyl of from 1 to 3 carbons, hydroxy, alkoxy of from 1 to 3 carbons $NH_2$, halogen, trifluoromethyl or nitro.

The activity of the peptides of the present invention as GRP antagonists was determined in competitive binding assays with a radioactive GRP derivative. Swiss 3T3 fibroblasts were used in these tests as the source of GRP receptor. Because these cells respond to GRP binding with a rapid increase in DNA synthesis, peptides that bind to the GRP receptor can also be tested for their ability to stimulate DNA synthesis. New DNA synthesis is one of the early steps in cell division and is widely accepted as a measure of mitogenicity or cell growth. Peptides which bind to the receptor and do not stimulate growth are then tested for their ability to block GRP stimulated DNA synthesis. Peptides which block DNA synthesis are mitogenic antagonists.

The peptides of the present invention can be synthesized from their constituent amino acids by conventional peptide synthesis techniques, preferably by solid phase technology. The peptides are then purified by reverse-phase high performance liquid chromatography (HPLC).

Standard methods of peptide synthesis are disclosed, for example, in the following works: Schroeder et al., "The Peptides", Vol. I, Academic Press 1965, or Bodanszky et al., "Peptide Synthesis", Interscience Publishers, 1966, or McOmie (ed.) "Protective Groups in Organic Chemistry", Plenum Press, 1973, or Barany et al., "The Peptides: Analysis, Synthesis, Biology" 2, Chapter 1, Academic Press, 1980. The teachings of these works are hereby incorporated by reference.

Known peptide antagonists of GRP are based on the structure of bombesin, a GRP analog containing 14 amino acids, or substance P, which contains 11 amino acids. The size of these antagonists are such that pharmacokinetic problems may be encountered. In addition, antagonists based on substance P show cross-reactivity with the substance P receptor.

Current chemotherapeutic agents for the treatment of SCLC are poorly effective. The treatment of SCLC by inhibiting the binding of GRP to its receptor offers advantages over conventional chemotherapy. First, use of a peptide antagonist avoids the gross toxic side affects of conventional chemotherapy. In addition, receptor antagonists do not need to enter the cell to be effective.

The peptides of the present invention are effective in inhibiting the growth of cells that are sensitive to the growth promoting activity of GRP.

The following examples illustrate the present invention without, however, limiting the same thereto. The abbreviation BSA stands for bovine serum albumin and the abbreviation DMEM stands for Dulbecco's Modified Eagle Medium.

## EXAMPLE 1

### Binding Inhibition Studies

Swiss 3T3 cells were obtained from Dr. K. Brown (Institute of Animal Physiology, Cambridge, U.K.). The cells were grown to confluency in Costar 12-well plates containing DMEM (Gibco) supplemented with 10% fetal bovine serum, 2 mM glutamine and 1% penicillin-streptomycin. The cells were washed twice with binding buffer [1:1 DMEM:Waymouths MB752/1 medium, plus 1 mg/ml BSA (Fraction V, Calbiochem)]. The antagonist was dissolved in 10 mM HCl, and diluted to the appropriate concentration in binding buffer. The antagonist was then added to the cells, followed by radiolabelled GRP at a final concentration of 7 nM. After 30 minutes incubation at 37°C, the supernatant liquid was removed and the cell monolayer rinsed four times with washing buffer (150 mM NaCl, 20 mM $Na_2HPO_4$, 5mM KCl, 1.8 mM $KH_2PO_4$, 1 mg/ml BSA). The cells were then lysed with 1 ml/well of lysis buffer (1% Triton X-100, 0.1% BSA), and the solution was aspirated into scintillation vials for counting. Each data point was collected in triplicate.

## EXAMPLE 2

Mitogenic Stimulation

Swiss 3T3 cells were grown in monolayer culture in 24-well plates (Costar) in serum-free DMEM for 48 hours, at which time the GRP or GRP homologue and 23 nM $^3$H thymidine were added. After an additional 48 hours, the cell monolayer was washed twice with PBS, and the cells were then removed with 1 ml 10x trypsin containing 5 mM EDTA. The cells were harvested with a Skatron filter apparatus, and the filters counted in a scintillation counter.

## EXAMPLE 3

Mitogenesis Inhibition

Swiss 3T3 cells were grown in monolayer culture in 24-well plates (Costar) in serum-free DMEM for 48 hours, at which time the GRP or a GRP homologue, the antagonist and 23 nM $^3$H-thymidine were added. After an additional 48 hours, the cell monolayer was washed twice with PBS, and the cells were then removed with 1 ml 10x trypsin containing 5 mM EDTA. The cells were harvested with a Skatron filter apparatus, and the filters counted in a scintillation counter. The following results were obtained.

| Peptide Antagonist | Binding Inhibition | EC$_{50}$* Mitogenic Stimulation | Mitogenic Inhibition |
|---|---|---|---|
| Ac-His-Trp-Ala-Val-Gly-His-LeuNH2 (Acetyl GRP 20-26 Amide) | 2 uM | >100 uM | 3 uM |
| His-Trp-Ala-Val-Gly-His-LeuNH2 (GRP 20-26 amide) | 20 uM | >100 uM | 50 uM |

*EC$_{50}$ Definitions:

Binding Inhibition: [Antagonist] which reduces the specific binding of 7 nM [$^3$H-Phe$^{15}$] GRP 15-27 to 50% of the level observed in the absence of antagonist. Assay performed in Swiss 3T3 cell monolayers.

Mitogenic Stimulation: [Agonist] which stimulates uptake of $^3$H-thymidine to 50% of the maximal level caused by [Nle$^{27}$] GRP 15-27. Assay performed on Swiss 3T3 monolayers.

Mitogenic Inhibition: [Antagonist] which reduces the uptake of $^3$H-thymidine caused by 7 nM [Nle$^{27}$] GRP 15-27 to 50% of the level observed in the absence of antagonist. Similar results obtained for the first antagonist using [Nle$^{27}$] GRP 1-27 as the mitogen. Assay performed on Swiss 3T3 monolayers.

## Claims

1. A peptide having the amino.acid sequence

$$\text{His} \quad \text{Trp} \quad \text{Ala} \quad \text{Val} \quad \text{Gly} \quad \text{His} \quad \text{Leu-NH}_2$$
$$\text{Ac-His} \quad \text{Trp} \quad \text{Ala} \quad \text{Val} \quad \text{Gly} \quad \text{His} \quad \text{Leu-NH}_2$$
$$\text{Ac-His} \quad \text{Trp} \quad \text{Ala} \quad \text{Val} \quad \text{Gly} \quad \text{His} \quad \text{Leu-NHR}^1$$
$$\text{Ac-His} \quad \text{Trp} \quad \text{Ala} \quad \text{Val} \quad \text{Gly} \quad \text{His} \quad \text{Leu-OR}^1$$

wherein R$^1$ is a straight or branched chain alkyl group of from 1 to 5 carbon atoms, or a phenyl group, either of which is optionally substituted by alkyl of from 1 to 3 carbon atoms, hydroxy, alkoxy of from 1 to 3 carbon atoms, NH$_2$, halogen, trifluoromethyl or nitro.

2. A SCLC cell having at least one peptide of Claim 1 bound to a GRP receptor on the surface of the cell.

3. A receptor ligand complex formed by binding a peptide of claim 1 to a GRP receptor of a SCLC cell.

4. A method of inhibiting the growth of cells that are sensitive to the growth promoting activity of GRP which comprises treating the cells with a peptide of claim 1 in an amount effective to antagonize the growth promoting activity of GRP.


Claims for the following Contracting State: ES

1.- A process for the preparation of a peptide, useful as a gastrin releasing peptide antagonist, having the following amino acid sequence:

$$\text{His} \quad \text{Trp} \quad \text{Ala} \quad \text{Val} \quad \text{Gly} \quad \text{His} \quad \text{Leu-NH}_2$$
$$\text{Ac-His} \quad \text{Trp} \quad \text{Ala} \quad \text{Val} \quad \text{Gly} \quad \text{His} \quad \text{Leu-NH}_2$$
$$\text{Ac-His} \quad \text{Trp} \quad \text{Ala} \quad \text{Val} \quad \text{Gly} \quad \text{His} \quad \text{Leu-NHR}^1$$
$$\text{Ac-His} \quad \text{Trp} \quad \text{Ala} \quad \text{Val} \quad \text{Gly} \quad \text{His} \quad \text{Leu-OR}^1$$

wherein $R^1$ is a straight or branched chain alkyl group of from 1 to 5 carbon atoms, or a phenyl group, either of which is optionally substituted by alkyl of from 1 to 3 carbon atoms, hydroxy, alkoxy of from 1 to 3 carbon atoms, $NH_2$, halogen, trifluoromethyl or nitro,
characterized by
-synthesizing the above mentioned amino acid sequences from their constituent amino acids by conventional peptide synthesis techniques, such as by solid-phase technology, followed by
-purifying them by reverse-phase high performance liquid chromatography.

2.- A process for the preparation of a receptor-ligand complex, characterized by binding a peptide obtained according to the process of claim 1 to a GRP receptor of a SCLC cell.

3.- A method of inhibiting the growth of cells that are sensitive to the growth promoting activity of GPR which comprises treating the cells obtained according to the process of claim 1 in an amount effective to antagonize the growth promoting activity of GRP.